(19) Europäisches Patentamt
European Patent Office
Office européen des brevets

(11) EP 4 188 309 B1

# (12) EUROPEAN PATENT SPECIFICATION

(45) Date of publication and mention of the grant of the patent:
**01.11.2023 Bulletin 2023/44**

(21) Application number: **21751538.6**

(22) Date of filing: **26.07.2021**

(51) International Patent Classification (IPC):
***A61K 8/02** (2006.01)* ***A61K 8/26** (2006.01)*
***A61K 8/29** (2006.01)* ***A61K 8/58** (2006.01)*
***A61Q 19/02** (2006.01)*

(52) Cooperative Patent Classification (CPC):
**A61K 8/26; A61K 8/0212; A61K 8/29; A61K 8/585; A61Q 19/02**

(86) International application number:
**PCT/EP2021/070871**

(87) International publication number:
**WO 2022/023276 (03.02.2022 Gazette 2022/05)**

## (54) COSMETIC MASK FOR IMPROVING APPEARANCE OF SKIN

KOSMETISCHE MASKE ZUR VERBESSERUNG DES HAUTBILDES

MASQUE COSMÉTIQUE POUR AMÉLIORER L'ASPECT DE LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.07.2020 PCT/CN2020/105518**
**27.08.2020 EP 20193194**

(43) Date of publication of application:
**07.06.2023 Bulletin 2023/23**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **Unilever Global IP Limited**
  **Wirral, Merseyside CH62 4ZD (GB)**
  Designated Contracting States:
  **CY DE GB IE IT MT RS TR**

(72) Inventors:
- **KULKARNI, Aditi,Jayavant**
  **Mumbai 400 099, Maharashtra (IN)**
- **MENG, Sheng**
  **Shanghai 200335 (CN)**
- **WASKAR, Princika**
  **Thane(W) 400 604, Maharashtra (IN)**

(74) Representative: **James, Helen Sarah**
**Unilever Patent Group**
**Bronland 14**
**6708 WH Wageningen (NL)**

(56) References cited:
**WO-A1-2020/109481** **US-A1- 2010 266 519**
**US-A1- 2018 371 257**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 188 309 B1

## Description

### Field of the invention

The present invention relates to cosmetic compositions for improving appearance of skin.
The present invention relates to a cosmetic composition, particularly for application to the face and neck. More particularly the composition is a face mask, in particular a clay-based rinse-off mask.

### Background of the invention

Conventionally, a cosmetic composition described as a mask is formulated to be applied to the skin in the form of a thick layer since it is meant for providing skin care, generally deep cleansing of the epidermis. After an application time required to accomplish this care, generally from 10 to 45 minutes, this layer is then removed by rinsing-off with water or alternatively by using a wet cloth or tissue.
Our skin is amenable to deterioration due to dermatological disorders, environmental factors such as strong sunlight and normal aging process (chrono-ageing), which may be accelerated by exposure to sun (photo-ageing). In recent years, the demand for cosmetic compositions and cosmetic methods for improving the appearance of skin has grown enormously. Consumers are increasingly seeking cosmetic compositions to treat, delay or conceal the visible signs of chrono-ageing and photo-ageing skin such as wrinkles, fine lines, sagging, hyperpigmentation and age spots.
WO2020109481 A1 (Unilever) discloses a clay mask composition having bentonite, oil, water, kaolin and thickener. The composition is devoid of surfactants and does not impart dry sensation after use.
WO19016493 A1 (Cosmetic Warriors Ltd) discloses a cosmetic composition comprising humectant; 25 to 80 % water; 15 to 50% of an inorganic material such as mica, zeolite, calamine and mixtures thereof and a gelling agent.
We have determined that cosmetic compositions according to the present invention surprisingly provide long-lasting improvement in the appearance of skin, especially facial skin.

### Summary of the invention

In accordance with a first aspect is disclosed a face mask cosmetic composition comprising:

i) 10 to 35 wt% of a clay; and
ii) 0.001 to 5 wt% of a skin benefit agent, and,
iii) 0.1 to 5 wt% titanium dioxide particles having primary particle size of 50 to 1000 nm and treated with a hydrophilic or hydrophobic agent;

wherein said composition comprises 0.05 to 5 wt% of a silicone resin and a solvent for the resin in an amount sufficient to solubilize the resin, where said resin is MQ silicone resin, T silicone resin or a mixture thereof; and wherein said solvent for the silicone resin is at least one of a volatile ester, volatile hydrocarbon, a volatile alcohol or a volatile silicone.

In accordance with a second aspect is disclosed use of a cosmetic composition of the first aspect for long-lasting skin brightening.
In accordance with as third aspect is disclosed a method of providing long-lasting skin brightening comprising applying a cosmetic composition of the first aspect to the skin.

### Detailed description

"Skin" as used herein, is meant to include skin on the face, neck, scalp, underarms, chest, back, arms, hands, legs, scalp, feet, buttocks and abdomen, preferably the hands, neck, face, and underarms.
Superficial imperfections of skin means and include blemishes (age spots, blotches), pores, fine lines and wrinkles, and grooves (the words "lines," "wrinkles," and grooves being used interchangeably herein). "Age spots" as used herein means any hyperpigmentation (e.g. including solar lentigo), spots and/or freckles.
The term treating or treatment as used herein includes within its scope reducing, delaying and/or preventing the abovementioned skin conditions and generally enhancing the quality of skin and improving its appearance and texture.
By "a cosmetic composition" as used herein, is meant to include a composition for topical application to the skin of mammals, especially human beings. Such a composition may be generally classified as leave-on or but is preferably rinse off.
Except in the examples, or where otherwise explicitly indicated, all numbers in this description indicating amounts

of material or conditions of reaction, physical properties of materials and/or use may optionally be understood as modified by the word "about". All amounts are by weight of the final composition, unless otherwise specified. The term "solid" as used herein means that the material is not fluid at 25ºC. It should be noted that in specifying any range of values, a given upper value can be associated with any lower value. For the avoidance of doubt, the word "comprising" is intended to mean "including" but not necessarily "consisting of" or "composed of'. In other words, the listed steps or options need not be exhaustive. The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

Mask, as used herein, means a paste-like material having a viscosity of 90,000 to 500,000 mPa.s (cps) (determined using RVT-F at 5 RPM, 1 minute, 25°C). The unit of viscosity, cps, used throughout this specification could be read in SI units as mPa.s (millipascal- second)

Clay

It is preferred that the composition comprises 10 to 20 wt% clay.

"Clay" means a naturally occurring material composed primarily of fine-grained minerals, which is generally plastic at appropriate water contents. Although clay usually contains phyllosilicates, it may contain other materials that impart plasticity and harden when dried or fired. Examples include Kaolin, Bentonite, Mite, Vermiculite, Smectite and Chlorite.

Reference may be made to Kirk-Othmer, Encyclopaedia of Chemical Technology, Volume 5, page 544, 2nd edition, John Wiley and Sons, Inc., New York, New York 1964. Clays may be of natural or synthetic origin. In one aspect, preferably the clay is hydrophilic. Alternatively, the clay is hydrophobic. Other examples of preferred clay include saponites, hectorites, montmorillonites, bentonites, laponites, Fuller's earth, montmorillonite, attapulgite, and palygorskite.

It is preferred that the clay is kaolin, bentonite or a combination of kaolin and bentonite.

It is preferred that the composition comprises 4 to 18 wt%, more preferably 6 to 16 wt% kaolin. In such a case, it is evident that the composition of the invention at least one more clay so that the minimum amount of 10 wt% can be met. Kaolin (or kaolinite) means a colloidal clay mineral having a chemical composition comprising $Al_2Si_2C_5(OH)_4$. Such a mineral is suitable to adhere to an oil and water interface of a clay mask. It is preferred that the kaolin is a colloidal particle of median particle size from 1 to 5 μm

It is preferred that the composition comprises 4 to 12 wt%, more preferably 4 to 8 wt% bentonite. In such a case, it is evident that the composition of the invention at least one more clay so that the minimum amount of 10 wt% can be met. The bentonite suitable for use in the composition of the present invention is for example, made commercially available from suppliers like H&H Clay, Inc., Wyoming Bentonite, Brenntag Specialties, Inc and Sigma Aldrich.

Preferably when the clay is a combination of kaolin and bentonite, the weight ratio of bentonite to kaolin in said composition is from 1:0.25 to 1:5 parts by weight. Further preferably this ratio is from 1:2 to 1:3 parts by weight.

Silicone resin

The cosmetic composition of the invention comprises 0.05 to 5 wt% of a silicone resin.

The resin is suitable for use in cosmetic composition and should be film-forming silicone resin. The silicone resin is typically described by the following siloxy monomeric units:

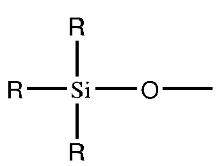

$M=(R)_3SiO_{1/2}$

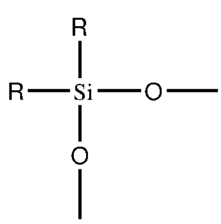

$D=(R)_2SiO_{2/2}$

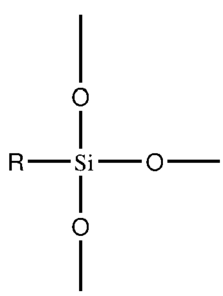

$T=RSiO_{3/2}$

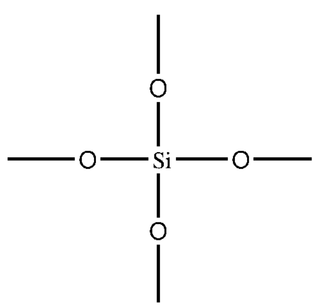

$Q=SiO_{4/2}$

.

The R group may be selected from saturated or unsaturated hydrocarbon groups. Preferably, the silicone resin is selected from siloxysilicate, silsesquioxane, or a mixture thereof. More preferably, the silicone resin comprises M unit, Q unit, T unit or combination thereof. Even more preferably, the silicone resin comprises MQ silicone resin, T silicone resin, or a mixture thereof.

In some embodiments, the silicone resin preferably comprises MQ silicone resin having the formula of $[(R_1)_3\text{-Si-}O_{1/2}]_a\text{-}(Si\text{-}O_{4/2})_b$, wherein $R_1$ is mutually identical or different, selected from saturated hydrocarbon groups. $R_1$ is preferably selected from $C_1$ to $C_6$ alkyl, and more preferably each $R_1$ is methyl group. Thus, the more preferred MQ silicone resin is trimethylsiloxysilicate. Preferably, a and b independently have values ranging from 10 to 1000, and more preferably from 30 to 200.

In other embodiments, the silicone resin preferably comprises T silicone resin having the formula of $[R_2\text{-Si-}O_{3/2}]_x$, wherein $R_2$ is selected from saturated hydrocarbon groups.

$R_2$ is preferably selected from $C_1$ to $C_6$ alkyl, more preferably selected from methyl, ethyl, propyl, butyl, and most preferably propyl. The most preferred T silicone resin is polypropyl silsesquioxane. Preferably, x is less than 2000, more preferably less than 500, but preferably greater than 10, and more preferably greater than 50.

The silicone resin preferably comprises a blend of MQ silicone resin and T silicone resin, the weight ratio of MQ silicone resin to T silicone resin is preferably from 1:20 to 20:1 in order to achieve better film-forming performance. More preferably, the weight ratio of MQ silicone resin to T silicone resin is from 1:10 to 10:1, even more preferably from 1:5 to 5:1.

In most preferred embodiments, the composition comprises MQ silicone resin. Preferably the MQ silicone resin is present in amount of at least 20% by weight of the total silicone resin, more preferably at least 40%, even more preferably at least 70%, and still even more preferably at least 85% by weight of the total silicone resin. Most preferably, MQ silicone resin is present in amount of 100% by weight of the total silicone resin.

Exemplary silicone resins suitable for the present invention includes Dow Corning™ MQ-1640 Flake Resin, a blend of MQ and T Propyl resins, Dow Corning™ MQ-1600 Solid Resin, a 100% active MQ resin, Dow Corning™ 670 Fluid, Cyclopentasiloxane (and) Polypropylsilsesquioxane supplied by Dow Corning.

It is preferred that the composition of the invention comprises 0.1 to 3 wt% silicone resin. Reference to wt% is based on the solids content. Preferably the silicone resin is selected from trimethylsiloxy silicate, polypropyl silsesquioxane, or a combination thereof. More preferably the silicone resin is mixture of trimethylsiloxy silicate and polypropyl silsesquioxane.

Solvent

It is preferred that the solvent for the silicone reason is at least one of a volatile ester, volatile hydrocarbon, a volatile alcohol or a volatile silicone.

When the solvent is a volatile ester it preferably is at least one of isopropyl myristate, isopropyl palmitate, dicaprylyl carbonate, capric/caprylic triglycerides or a C12-15 alkyl benzoate.

When the solvent is a hydrocarbon it preferably is at least one of C11-13 isoparaffin, isodecane, C13-15 alkane, isohexadecane.

When the solvent is an alcohol it preferably is ethanol.

When the solvent is a silicone it preferably is at least one of cyclopentasiloxane such as XIAMETER PMX-0245, dimethyl polysiloxane of viscosity 1 to 10 cSt, such as XIAMETER PMX-200 silicone fluid, polyphenylmethylsiloxane (example DOWSIL™ 556 cosmetic grade fluid) or volatile alkyl methyl siloxane, such as caprylyl branched trisiloxane (example DOWSIL™ FZ-3196 fluid)

The solvent is present in an amount proportionate to the corresponding amount of the resin, in an amount sufficient to solubilize the resin. Some of the commercial silicone resins example, Dow Corning™ 670 fluid, already contain a solvent.

It is preferred that the solvent is a silicone, especially cyclopentasiloxane.

Titanium dioxide

The composition of the invention comprises 0.1 to 5 wt% titanium dioxide particles having primary particle size of 50 to 1000 nm and treated with at least one of a hydrophilic or hydrophobic agent. Further preferably the titanium oxide particles are treated with a hydrophilic and a hydrophobic agent.

Further preferably, the hydrophilic agent is aluminium oxide, aluminium hydroxide or aluminium silicate and said hydrophobic agent is aluminium stearate, stearic acid, aluminium laurate, zinc stearate isostearic acid, lauroyl lysine, or dimethicone. The particle size may be measured by Mac-View particle size distribution (electron microscopy) method.

It is preferred that the particle size is from 50 to 250 nm and that the particles are treated with stearic acid (and) aluminum hydroxide. An example of such an ingredient is MT-700Z ex. Tayca Corporation having particle size of 80 nm.

Alternatively, and preferably the titanium oxide particles having primary particle size of 50 to 1000 nm is selected

from the group consisting of:

| Trade name | Agent(s) | Particle size/nm |
|---|---|---|
| MTY700BS | hydrogen dimethicone | 80 |
| MPY1133EX | isostearic acid | 250 |
| MPY25S | hydrogen dimethicone | 250 |
| MPY1133S | hydrogen dimethicone | 250 |
| MPY1133M | dimethicone | 250 |
| MPY1133LL | lauroyl lysine | 250 |
| MPY100EX | isostearic acid | 1000 |
| MPY100M | aluminium hydroxide (and) dimethicone (and) zinc oxide | 1000 |
| $TiO_2$ silicon dispersion | cyclopentasiloxane (and) dimethicone (and) PEG-10 dimethicone (and) alumina | 215 to 255 |

Skin benefit agent

It is preferred that the skin benefit agent provides at least one of glow, even tone, skin clarity or radiance to skin.

Preferably the composition comprises 0.1 to 3 wt% of skin benefit agent, the wt% range applying to all and any of the benefit agents.

Preferably the skin benefit agent is at least one of retinol, retinyl ester, resorcinol, niacin, nicotinic acid, niacinamide, allantoin, ubiquinone, conjugated linoleic acid, 12-hydroxystearic acid or a 4-alkyl resorcinol.

The term "retinol" includes the following isomers of retinol: all-trans-retinol, 13-cis-retinol, 11-cis-retinol, 9-cis-retinol, 3,4-didehydro-retinol. Preferred isomers are all-trans-retinol, 13-cis-retinol, 3,4-didehydro-retinol, 9-cis-retinol. Most preferred is all-trans-retinol, due to its wide commercial availability.

Retinyl ester is an ester of retinol. Retinyl esters suitable for use in the present invention are C1-C30 esters of retinol, preferably C2-C20 esters, and most preferably C2, C3, and C16 esters because they are more commonly available. Examples of retinyl esters include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecandate, retinyl laurate, retinyl tridecanoate, retinyl myristate, retinyl pentadecanoate, retinyl heptadeconoate, retinyl stearate, retinyl isostearate, retinyl nonadecanoate, retinyl arachidonate, retinyl behenate, retinyl linoleate, retinyl oleate, retinyl lactate, retinyl glycolate, retinyl hydroxy caprylate, retinyl hydroxy laurate, refinyl tartarate. The preferred ester for use in the present invention is selected from retinyl palmitate, retinyl acetate and retinyl propionate, because these are efficacious while being most commercially available and therefore the cheapest.

Resorcinols and its derivatives have a wide variety of applications including use in cosmetic products. Resorcinols are reported to have antioxidant, sun-protective, anti-acne, antiseptic and other therapeutic properties. In particular, 4-alkyl resorcinol is reported to have skin-beautifying effect and skin brightening benefits. Especially preferred resorcinol for use in the present invention are 4-substituted resorcinol. Most preferred 4-substituted resorcinol are 4-alkyl resorcinol including 4-ethyl resorcinol, 4-hexyl resorcinol, 4-butyl resorcinol, or 4-phenethyl resorcinol.

Vitamin B3 compounds have been known as skin brightening agents for a long time. They have also been reported to have various other properties beneficial to the skin. Vitamin B3 compound or its derivative include niacin, nicotinic acid, and niacinamide most preferred being niacinamide.

Conjugated linoleic acid (CLA) has been known for the effective treatment and prevention of normal skin conditions due to aging or exposure to light and solar radiation, such as wrinkles, lines, sagging, hyperpigmentation and age spots. Conjugated linoleic acid comprises a group of positional and geometric isomers of linoleic acid in which various configurations of cis and trans double bonds at positions (6, 8), (7, 9), (8, 10), (9, 11), (10, 12) or (11, 13) are possible. Each positional isomer can have 4 geometrical isomers. For example in the case of the (6,8) isomer, there can be cis-6-cis-8, cis-6-trans-8, trans-6-cis-8 and trans-6-trans-8 geometrical isomers. Similarly there can be 4 geometrical isomers for the remaining 5 positional isomers. Thus twenty- four different isomers of the CLA can exist. CLA is different from "linoleic acid". Linoleic acid is a straight chain fatty acid, chemically referred to as cis-9, cis-12-octadecadienoic acid. In the case of linoleic acid the two double bonds are not in conjugation.

Ubiquinone is also known as Coenzyme Q10, it is a 1 ,4-benzoquinone, where Q refers to the quinone chemical group, and 10 refers to the number of isoprenyl chemical subunits in its tail. It inhibits both the initiation and the propagation

of lipid and protein oxidation. It also regenerates other antioxidants such as vitamin E. In cosmetic product, ubiquinone is used as antioxidant.

Allantoin is called 5-ureidohydantoin or glyoxyldiureide. It is a diureide of glyoxylic acid. It is frequently present in lipsticks, cosmetic lotions and creams, and used as antioxidant due to the oxidation of uric acid by purine catabolism.

12-hydroxystearic acid (generally referred to as "12-HSA") is reported to have a wide variety of beneficial cosmetic effects on skin, e.g. it is a known as a PPAR-alpha (peroxisome proliferator activated receptors sub-type alpha) activator, skin brightening agent, and a sebum secretion inhibitor. 12-HSA has also traditionally been used as gelling agent e.g. in lipsticks and anti-perspirant compositions. 12-HSA has also been used to increase viscosity of cosmetic compositions.

It is preferred that viscosity of cosmetic composition is from 90,000 to 500,000 cps (determined using RVT-F at 5 RPM, 1 minute, 25°C). More preferably the viscosity is from 100,000 to 300,000 cps, and still more preferably from 100,000 cps to 200,000 cps, including all ranges subsumed therein.

It is particularly preferred that the cosmetic composition of the invention is a cosmetic face mask.

In one aspect the face mask is to be used by direct application to the face. Alternatively, and preferably the face mask is a product that comprises a water insoluble porous substrate where the composition of the invention is impregnated in the substrate. In such a case, the water insoluble porous substrate is preferably selected from paper, polymeric web, fabric, or composites/mixtures thereof. The porous substrate is preferably paper or fabric. The porous substrate is preferably configured as discrete sheets, a bundle of such sheets, preferably packed together as a bunch for sale to the consumer. The area of each such sheet is preferably from 100 to 1000 $cm^2$, preferably 300 to 500 $cm^2$, more preferably 350 to 450 $cm^2$. The porous substrate in a further preferred aspect is configured in the shape that can be conveniently applied on the face of an individual with holes for the nose, mouth and eyes.

Further preferably the cosmetic composition of the invention comprises a scrubbing, exfoliating or abrasive agent. Preferably this agent is not plastic or microplastic. Preferably the agent is walnut shell particles, sugar, salt, seeds, pumice, oat or other ingredients cosmetically acceptable to be used for such purpose.

Nonvolatile polyalkyl siloxanes useful herein include, for example, polydimethyl siloxanes.

Where the cosmetic compositions of the invention comprise an organopolysiloxane crosspolymer, the polymer preferably is dimethicone/vinyl dimethicone crosspolymer or dimethicone crosspolymer available from a variety of suppliers including Dow Corning (9040, 9041, 9045, 9506 and 9509), General Electric (SFE 839), Shin Etsu (KSG-15, 16 and 18 [dimethicone/phenyl vinyl dimethicone crosspolymer]), and Grant Industries (Gransil brand of materials), and lauryl dimethicone/vinyl dimethicone crosspolymers supplied by Shin Etsu (e.g. KSG-31, KSG-32, KSG-41, KSG-42, KSG-43 and KSG-44).

Where the compositions comprise non-silicone emollient, the emollient preferably is stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate, and mixtures thereof.

Among the ester emollients are alkenyl or alkyl esters of fatty acids having 10 to 20 carbon atoms. Examples thereof include isoarachidyl neopentanoate, isodecyl neopentanoate, isononyl isonanoate, cetyl ricinoleate, oleyl myristate, oleyl stearate, and oleyl oleate; ether-esters such as fatty acid esters of ethoxylated fatty alcohols; polyhydric alcohol esters, butylene glycol, ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C1 to 30 alcohols. Exemplative is pentaerythrityl tetraethylhexanoate; wax esters such as beeswax, spermaceti wax and tribehenin wax; sterols esters, of which cholesterol fatty acid esters are examples thereof; sugar ester of fatty acids such as sucrose polybehenate and sucrose polycottonseedate; or mixtures of two or more of the foregoing (a) to (f).

Hydrocarbons which are suitable emollients include petrolatum, mineral oil, C11-C13 isoparaffins, polyalphaolefins, isohexadecane or a mixture thereof.

Preferably the cosmetic compositions of the invention comprise 20 to 80 wt% water, more preferably 40 to 75% water. This water forms part of the carrier.

Humectants include those of the polyhydric alcohol-type. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, glycerol, ethoxylated glycerol, propoxylated glycerol and mixtures thereof. The amount of humectant may range, for example, anywhere from 0.5 to 50 wt%, more preferably between 1 and 15% by weight of the composition. Most preferred is glycerol (also known as glycerin). Amounts of glycerin may range, for example, from 0.5 to 50 wt%, more preferably from 1 to 35%, optimally from 2 to 15 wt%.

A variety of thickening agents may be included in the compositions. Illustrative but not limiting are stearic acid, Acrylamide/Sodium Acryloyldimethyltaurate Copolymer (Aristoflex AVC), Hydroxyethyl Acrylate/Sodium Acryloyldimethyltau rate Copolymer, Aluminum Starch Octenyl Succinate, Polyacrylates (such as Carbomers including Carbopol® 980, Carbopol® 1342, Pemulen® TR-2 and the Ultrez® thickeners, Polysaccharides (including xanthan gum, guar gum, pectin, carageenan and sclerotium gums), celluloses (including carboxymethyl cellulose, ethyl cellulose, hydroxyethyl cellulose and methyl hydroxymethyl cellulose), minerals (including talc, silica, alumina, mica and clays, the latter being represented by bentonites, hectorites and attapulgites), magnesium aluminum silicate and mixtures thereof. Amounts of the thickeners may range, for example, from 0.05 to 10%, more preferably from 0.3 to 5% by weight of the composition.

Powders include chalk, talc, starch, gums, carboxyvinyl polymer, sodium carboxymethyl cellulose and ethylene glycol monostearate.

Preferably, the composition comprises a further pigment, other than titanium dioxide particles having primary particle size of 50 to 1000 nm. The pigments are typically particles of high refractive index materials. For example, the pigment may have a refractive index of greater than 1 .3, more preferably greater than 1 .8 and most preferably from 2.0 to 2.7. Examples of such pigment are those comprising bismuth oxy-chloride barium sulfate, mica, silica, titanium dioxide, zirconium oxide, aluminum oxide, zinc oxide or combinations thereof. More preferred pigment are particles comprising titanium dioxide, zinc oxide, zirconium oxide, mica, iron oxide or a combination thereof. Even more preferred pigment are particles comprising zinc oxide, zirconium oxide, titanium dioxide or a combination thereof as these materials have especially high refractive index. Still even more preferably the pigment is selected from titanium dioxide, zinc oxide or a mixture thereof and most preferred whitening pigment is titanium dioxide.

Preferably the composition comprises 0.001 to 10 wt% pigment, more preferably 0.01 to 6 wt%, more preferably still 0.1 to 3 wt% and most preferably 0.2 to 2 wt% pigment.

The cosmetic compositions of the invention may further comprise other ingredients which are common in the art to enhance physical properties and performances.

Optional ingredients suitable for use in the composition of the present invention include extracts like chamomile, green tea, liquorice, grape seed, sage, aloe vera, thyme, rosemary mixtures thereof or the like. Other ingredients include humectants like glycerine and/or sorbitol, vitamins such as vitamin C as well as dihydroxyacetone, and sunscreens like benzophenone-4 and phenylbenzimidazole sulphonic acid, Vitamin A (and its oil soluble derivatives), D, E (and its oil soluble derivatives) and K, sunscreens like methoxycinnamate, octyl methoxycinnamate, bis-ethyl hexyloxyphenol methoxyphenyl triazine, drometrizole trisiloxane or mixtures thereof.

When present, such ingredients may be present in an amount falling in the range of 0.01 to 10 wt%.

Suitable traditional preservatives for use in this invention are alkyl esters of para-hydroxybenzoic acid. Other preservatives include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, 1 ,2-octanediol, hydroxyacetophenone, ethylhexylglycerine, hexylene glycol, methyl paraben, propyl paraben, imidazolidinyl urea, sodium dehydroacetate and benzyl alcohol. The preservatives should be selected having regard for the use of the composition and possible incompatibilities between the preservatives and other ingredients in the composition. Preservatives are preferably employed in amounts ranging from 0.01% to 2.5% by weight of the total weight of the composition, including all ranges subsumed therein. Combinations of 1 ,2-octanediol and phenoxyethanol, or iodopropynyl butyl carbamate and phenoxyethanol are preferred, with phenoxyethanol and 1 ,2-octanediol, collectively and preferably, making up less than 2% by weight of the total weight of the composition of the present invention. Also preferred is a preservative system with hydroxyacetophenone alone or in a mixture with other preservatives.

In order to stabilize certain oils and benefit agents, it is within the scope of the present invention to use antioxidants that are suitable for cosmetic compositions. Illustrative example of the antioxidants suitable for use in the present invention are Tinogard DA, Tinogard TT (commercially available from BASF), butylated hydroxytoluene, propyl gallate, tert-butyl hydoquinone mixture thereof or the like. When used, antioxidant makes up from about 0.001 to 1.0% by weight of the total weight of the composition.

The composition of the present invention is preferably prepared by mixing the ingredients with moderate shear (typically with a propeller blade) under atmospheric conditions and ambient temperature. Heating is optional if solid ingredients are present. Mixing is stopped after obtaining a homogeneous composition which preferably is homogenized using a Silverson Homogenizer until a clay mask composition having a viscosity from 90,000 to 500,000 cps is recovered. When heating is desired, the temperature of the composition is raised from ambient to 35 to 50°C, including all ranges subsumed therein.

Packaging for the composition of this invention can be a jar or tube as well as any other format typically seen for cosmetic, cream, washing and lotion type products. The compositions may be applied topically and preferably 1 to 4 milligrams of composition is applied per square centimeter of skin.

Use and Method

In accordance with a second aspect is disclosed use of the cosmetic composition for long-lasting skin brightening. Preferably a thin film of said composition is applied as a face mask and rinsed-off with water after leaving it on the face for 10 to 45 minutes. It is preferred that the effect of skin brightening is perceivable after 4 to 20 hours of application. It is further preferred that the extent of skin brightening after 4 to 20 hours of application is within 80 to 100% of the effect immediately after washing said face. It is further preferred that the extent of skin brightening is measured as herein described using a digital non-contact colour measurement tool.

For assessing color stability, color change (ΔE) is calculated based on the color measurement presented by Hunter lab color space L* a* and b* where, L* is black-white space, a* is green-red space, b* is blue-yellow space. For example, larger L* value means whiter, and smaller b* value means more blue.

Images of water solutions mentioned above were captured at time zero, i.e. just after they are made and at the end of the storage period, i.e. at the end of 4 weeks. All images were converted to L*, a* and b* data using Hunter lab-lab scan XE instrument. The values of L*, a* and b* at week 4 and week zero (initial data) were used to calculate ΔE using the formula:

$$\Delta E=\surd\{\text{square root over }((L_2{*}-L_1{*})+(a_2{*}-a_1{*})^2+(b_2{*}-b_1{*})^2)\}$$

When it is used as a face mask, application of the product may be effected as for any conventional face mask. For example, once a suitable size portion of the product has been taken from the composition, said portion is then applied to the skin of the user. Optionally, said portion may be mixed with a bit of water and then applied to the skin of the user. The applied composition may then be allowed to dry and left on the skin typically for up to about half an hour. The product is then washed off the skin. This also provides a pleasant soothing sensation for the user upon washing the product off their skin.

Therefore it is preferred that the composition of the invention provides long-lasting skin brightening, wherein the extent of skin brightening is measured as herein described using a digital non-contact colour measurement tool.

In accordance with another aspect is disclosed a method of providing long-lasting skin brightening comprising applying a cosmetic composition of the first aspect to the skin. Preferably the composition is applied as a face mask and rinsed-off with water after leaving it on the face for 10 to 45 minutes. Further preferably the skin brightening is perceivable after 4 to 20 hours of application. Yet further preferably the extent of skin brightening after 4 to 20 hours of application is within 80 to 100% of the effect immediately after washing said face.

All other aspects of the present invention will more readily become apparent upon considering the detailed description and examples which follow.

The following examples are provided to facilitate an understanding of the invention. The examples are not intended to limit the scope of the claims.

## Examples

Example 1

The compositions are described in Table 1. Composition C was control and Composition 1 was as per the present invention.

Table 1

| **Ingredients/wt%** | **Composition C** | **Composition 1** |
|---|---|---|
| Glycerine | 6.0 | 6.0 |
| Xanthum Gum | 0.4 | 0.4 |
| Anatase | 3.0 | 3.0 |
| Stearic acid | 2.2 | 2.2 |
| Cetostearyl alcohol | 1.0 | 1.0 |

(continued)

| Ingredients/wt% | Composition C | Composition 1 |
|---|---|---|
| Kaolin | 15.0 | 12.0 |
| PEG-400 | 4.0 | 4.0 |
| Bentonite | 6.0 | 5.0 |
| Niacinamide | 1.0 | 1.0 |
| SLES (28%) | 2.0 | 2.0 |
| DC 245 | - | 2.0 |
| MQ 1640 | - | 2.0 |
| MT700Z | - | 2.0 |
| Water and other minors | To 100 wt% | To 100 wt% |

The ΔE* values were measured as described below and summarised in Table 2. The ΔE* value is an indicator of brightening and brightening in turn connotes glowing skin.

The clay mask was applied on 50# Plain Bio-skin plate. The dosage was 2mg/cm$^2$. The composition was allowed to dry for 30 minutes after which a photograph was taken using DigiEye®. The L (for lightness), a, and b (for the color-opponent dimensions) of these coated Bio- skin plates was measured using the DigiEye Imaging System (Verivide, UK). Then the plates were washed by a washing machine to simulate the normal steps that a person would take to wash his/her face. The plates were then dried under air flow, after which a photograph was clicked again. After allowing the plates to dry for 20 hours a photograph was clicked again.

Colour of the skin was measured from the images and expressed as the CIE L*a*b* values. L* represent the lightness (100 = white, 0= black), a* is the green (-ve values) to red axis (+ve values) and b* is the blue (-ve values) to yellow axis (+ve values). The change in lightness and red-green colour before and after application of the cream was calculated as: L* = L(before) - L(after) • a = a(after) - a(before)

ΔE* values were measured as:

$$\Delta E *_{ab} = \sqrt{\left(L_1^* - L_2^*\right)^2 + \left(a_1^* - a_2^*\right)^2 + \left(b_1^* - b_2^*\right)^2}$$

Table 2

| ΔE* | Composition C | Composition 1 |
|---|---|---|
| After 30 minutes of application (before wash) | 20 | 25 |
| Immediately after wash | 1 | 4 |
| 20 hours after wash | 2 | 4 |

The data indicates that after:

a) at 30 minutes after application, the ΔE* of Composition 1 was 5 points more than the comparative composition.
b) immediately after washing the plates, the ΔE* Composition 1 was much higher than the comparative composition
c) after 20 hours of washing the plates, the ΔE* of Composition 1 remained the same as the value immediately after wash.

Therefore Composition 1 was much more efficacious in terms of skin brightening or glowing skin, as compared to the comparative composition.

## Claims

1. A face mask cosmetic composition comprising:

   i) 10 to 35 wt% of a clay; and
   ii) 0.001 to 5 wt% of a skin benefit agent, and,
   iii) 0.1 to 5 wt% titanium dioxide particles having primary particle size of 50 to 1000 nm as measured by Mac-View particle size distribution (electron microscopy) method; and treated with at least one of a hydrophilic or hydrophobic agent;
   wherein said composition comprises 0.05 to 5 wt% of a silicone resin and a solvent for the resin in an amount sufficient to solubilize the resin, where said resin is MQ silicone resin, T silicone resin or a mixture thereof;
   wherein said solvent for the silicone resin is at least one of a volatile ester, volatile hydrocarbon, a volatile alcohol or a volatile silicone.

2. A cosmetic composition as claimed in claim 1 wherein particle size of said titanium dioxide particles is 50 to 250 nm.

3. A cosmetic composition as claimed in claim 1 or 2 wherein said hydrophilic agent is aluminium oxide, aluminium hydroxide or aluminium silicate and said hydrophobic agent is aluminium stearate, aluminium laurate, stearic acid, zinc stearate isostearic acid, lauroyl lysine or dimethicone.

4. A cosmetic composition as claimed in any of claims 1 to 3 wherein said clay is kaolin, bentonite or a combination of kaolin and bentonite.

5. A cosmetic composition as claimed in claim 4 wherein when said clay is a combination of kaolin and bentonite, the weight ratio of bentonite to kaolin is from 1 :0.25 to 1:5 parts by weight.

6. A cosmetic composition as claimed in claim 5 wherein said ratio is from 1:2 to 1:3 parts by weight.

7. A cosmetic composition as claimed in any of claims 1 to 6 wherein said skin benefit agent provides at least one of glow, even tone, skin clarity or radiance to skin.

8. A cosmetic composition as claimed in claim 7 wherein said skin benefit agent is at least one of retinol, retinyl ester, resorcinol, niacin, nicotinic acid, niacinamide, allantoin, ubiquinone, conjugated linoleic acid, 12-hydroxystearic acid or a 4-alkyl resorcinol.

9. A cosmetic composition as claimed in claim 8 wherein said skin benefit agent is niacinamide.

10. A cosmetic composition as claimed in any of claims to 1 to 9 wherein viscosity of the composition is from 90,000 to 500,000 mPa.s (cps) (determined using RVT-F at 5 RPM, 1 minute, 25°C).

11. A cosmetic composition as claimed in any of claim 1 to 10 wherein said composition provides long-lasting skin brightening, wherein the extent of skin brightening is measured as herein described using a digital non-contact colour measurement tool.

12. Use of a cosmetic composition as claimed in any of claims 1 to 11 for long-lasting skin brightening.

13. A method of providing long-lasting skin brightening comprising applying a cosmetic composition as claimed in any of claims 1 to 11 to the skin.

## Patentansprüche

1. Kosmetische Zusammensetzung für Gesichtsmasken, umfassend:

   i) 10 bis 35 Gew.-% eines Tons; und
   ii) 0,001 bis 5 Gew.-% eines Hautpflegemittels, und
   iii) 0,1 bis 5 Gew.-% Titandioxidpartikel mit einer Primärpartikelgröße von 50 bis 1000 nm, gemessen mit dem Mac-View-Partikelgrößenverteilungs (Elektronenmikroskopie)-Verfahren und behandelt mit mindestens einem

hydrophilen oder hydrophoben Mittel;

wobei die Zusammensetzung 0,05 bis 5 Gew.-% eines Silikonharzes und ein Lösungsmittel für das Harz in einer ausreichenden Menge umfasst, um das Harz aufzulösen, wobei das Harz ein MQ-Silikonharz, T-Silikonharz oder eine Mischung davon ist, wobei das Lösungsmittel für das Silikonharz mindestens eines von einem flüchtigen Ester, flüchtigen Kohlenwasserstoff, einem flüchtigen Alkohol oder einem flüchtigen Silikon ist.

2. Kosmetische Zusammensetzung, wie im Anspruch 1 beansprucht, wobei die Partikelgröße der Titandioxidpartikel 50 bis 250 nm beträgt.

3. Kosmetische Zusammensetzung, wie im Anspruch 1 oder 2 beansprucht, wobei das hydrophile Mittel Aluminiumoxid, Aluminiumhydroxid oder Aluminiumsilikat und das hydrophobe Mittel Aluminiumstearat, Aluminiumlaurat, Stearinsäure, Zinkstearat, Isostearinsäure, Lauroyllysin oder Dimethicon ist.

4. Kosmetische Zusammensetzung, wie in einem der Ansprüche 1 bis 3 beansprucht, wobei der Ton Kaolin, Bentonit oder eine Kombination von Kaolin und Bentonit ist.

5. Kosmetische Zusammensetzung, wie im Anspruch 4 beansprucht, wobei dann, wenn der Ton eine Kombination von Kaolin und Bentonit ist, das Gewichtsverhältnis von Bentonit zu Kaolin 1:0,25 bis 1:5 Gewichtsteile beträgt.

6. Kosmetische Zusammensetzung, wie im Anspruch 5 beansprucht, wobei das Verhältnis 1:2 bis 1:3 Gewichtsteile beträgt.

7. Kosmetische Zusammensetzung, wie in einem der Ansprüche 1 bis 6 beansprucht, wobei das Hautpflegemittel der Haut mindestens Glanz, gleichmäßigen Ton, Hautreinheit oder ein strahlendes Aussehen verleiht.

8. Kosmetische Zusammensetzung, wie im Anspruch 7 beansprucht, wobei das Hautpflegemittel mindestens eines von Retinol, Retinylester, Resorcin, Niacin, Nicotinsäure, Niacinamid, Allantoin, Ubichinon, konjugierter Linolsäure, 12-Hydroxystearinsäure oder einem 4-Alkylresorcin ist.

9. Kosmetische Zusammensetzung, wie im Anspruch 8 beansprucht, wobei das Hautpflegemittel Niacinamid ist.

10. Kosmetische Zusammensetzung, wie in einem der Ansprüche 1 bis 9 beansprucht, wobei die Viskosität der Zusammensetzung 90.000 bis 500.000 mPa.s (cps) beträgt (bestimmt unter Verwendung von RVT-F bei 5 U/min, 1 Minute, 25°C).

11. Kosmetische Zusammensetzung, wie in einem der Ansprüche 1 bis 10 beansprucht, wobei die Zusammensetzung eine lang anhaltende Hautaufhellung bewirkt, wobei das Ausmaß der Hautaufhellung, wie hier beschrieben, unter Verwendung eines digitalen berührungslosen Farbmessinstruments gemessen wird.

12. Verwendung einer kosmetischen Zusammensetzung, wie in einem der Ansprüche 1 bis 11 beansprucht, zur langanhaltenden Hautaufhellung.

13. Verfahren zum Bewirken einer langanhaltenden Hautaufhellung, umfassend das Auftragen einer kosmetischen Zusammensetzung, wie in einem der Ansprüche 1 bis 11 beansprucht, auf die Haut.

**Revendications**

1. Composition cosmétique de masque facial comprenant :

i) 10 à 35 % en poids d’une argile ; et
ii) 0,001 à 5 % en poids d’un agent bénéfique pour la peau, et
iii) 0,1 à 5 % en poids de particules de dioxyde de titane ayant une granulométrie primaire de 50 à 1000 nm, telle que mesurée par le procédé de distribution de granulométrie Mac-View (microscopie électronique) ; et traitées avec au moins l’un parmi un agent hydrophile et un agent hydrophobe ;
laquelle composition comprend 0,05 à 5 % en poids d’une résine de silicone et un solvant pour la résine en une quantité suffisante pour solubiliser la résine, où ladite résine est une résine de silicone MQ, une résine de

silicone T ou un mélange de celles-ci ;
dans laquelle ledit solvant pour la résine de silicone est au moins l'un parmi un ester volatil, un hydrocarbure volatil, un alcool volatil, et une silicone volatile.

**2.** Composition cosmétique selon la revendication 1, dans laquelle la granulométrie desdites particules de dioxyde de titane est de 50 à 250 nm.

**3.** Composition cosmétique selon la revendication 1 ou 2, dans laquelle ledit agent hydrophile est l'oxyde d'aluminium, l'hydroxyde d'aluminium ou le silicate d'aluminium et ledit agent hydrophobe est le stéarate d'aluminium, le laurate d'aluminium, l'acide stéarique, le stéarate de zinc, l'acide isostéarique, la lauroyl-lysine, ou la diméthicone.

**4.** Composition cosmétique selon l'une quelconque des revendications 1 à 3, dans laquelle ladite argile est le kaolin, la bentonite, ou une combinaison de kaolin et de bentonite.

**5.** Composition cosmétique selon la revendication 4, dans laquelle, quand ladite argile est une combinaison de kaolin et de bentonite, le rapport en poids de la bentonite au kaolin est de 1/0,25 à 1/5 parties en poids.

**6.** Composition cosmétique selon la revendication 5, dans laquelle ledit rapport est de 1/2 à 1/3 parties en poids.

**7.** Composition cosmétique selon l'une quelconque des revendications 1 à 6, dans laquelle ledit agent bénéfique pour la peau apporte à la peau au moins l'un parmi l'éclat, l'uniformité du teint, la clarté de la peau, et la luminosité.

**8.** Composition cosmétique selon la revendication 7, dans laquelle ledit agent bénéfique pour la peau est au moins l'un parmi le rétinol, un ester rétinylique, le résorcinol, la niacine, l'acide nicotinique, le niacinamide, l'allantoïne, l'ubiquinone, un acide linoléique conjugué, l'acide 12-hydroxystéarique, et un 4-alkyl-résorcinol.

**9.** Composition cosmétique selon la revendication 8, dans laquelle ledit agent bénéfique pour la peau est le niacinamide.

**10.** Composition cosmétique selon l'une quelconque des revendications 1 à 9, dans laquelle la viscosité de la composition est de 90 000 à 500 000 mPa.s (cps) (déterminée par utilisation de RVT-F à 5 t/min, 1 minute, 25°C.

**11.** Composition cosmétique selon l'une quelconque des revendications 1 à 10, laquelle composition offre un éclaircissement de la peau sur une longue période, dans laquelle l'ampleur de l'éclaircissement de la peau est mesurée comme décrit ici par utilisation d'un outil de mesure des couleurs sans contact numérique.

**12.** Utilisation d'une composition cosmétique selon l'une quelconque des revendications 1 à 11 pour éclaircir la peau sur une longue période.

**13.** Procédé pour réaliser un éclaircissement de la peau sur une longue période, comprenant l'application sur la peau d'une composition cosmétique selon l'une quelconque des revendications 1 à 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020109481 A1
- WO 19016493 A1


### Non-patent literature cited in the description


- **KIRK-OTHMER.** Encyclopaedia of Chemical Technology. John Wiley and Sons, Inc, 1964, vol. 5, 544